# EUROPEAN PATENT SPECIFICATION

(11) **EP 2 495 320 B1**
(45) Date of publication and mention of the grant of the patent: **27.02.2019**
(21) Application number: 10826636.2
(22) Date of filing: 22.10.2010
(51) Int. Cl.: C12N 15/09, C12N 5/10

(54) **METHOD FOR INDUCTION OF DIFFERENTIATION OF STEM CELLS INTO HEPATOCYTES**
VERFAHREN ZUR INDUKTION VON STAMMZELLDIFFERENZIERUNG IN HEPATOZYTEN
PROCÉDÉ PERMETTANT D'INDUIRE LA DIFFÉRENCIATION DES CELLULES SOUCHES EN HÉPATOCYTES

(30) Priority: 28.10.2009 JP 2009247342; 27.05.2010 JP 2010121282; 06.07.2010 JP 2010154225
(43) Date of publication of application: 05.09.2012
(73) Proprietor: Japan Health Sciences Foundation, Tokyo 1010032 (JP)
(72) Inventor: MIZUGUCHI, Hiroyuki, Ibaraki-shi Osaka 567-0085 (JP); KAWABATA, Kenji, Ibaraki-shi Osaka 567-0085 (JP); INAMURA, Mitsuru, Ibaraki-shi Osaka 567-0085 (JP); FURUE, Miho, Ibaraki-shi Osaka 567-0085 (JP)
(74) Representative: Zwicker, Jörk
(86) International application number: PCT/JP2010/068703
(87) International publication number: WO 2011/052504

(56) References cited:
- US-A1- 2005 037 493
- SUETSUGU ATSUSHI ET AL: "Differentiation of mouse hepatic progenitor cells induced by hepatocyte nuclear factor-4 and cell transplantation in mice with liver fibrosis", TRANSPLANTATION,, vol. 86, no. 9, 15 November 2008 (2008-11-15), pages 1178-1186, XP009136906,
- SEGUIN CHERYLE A ET AL: "Establishment of endoderm progenitors by SOX transcription factor expression in human embryonic stem cells", CELL STEM CELL, CELL PRESS, US, vol. 3, no. 2, 1 August 2008 (2008-08-01), pages 182-195, XP009108846, ISSN: 1934-5909, DOI: 10.1016/J.STEM.2008.06.018
- XIAO LING KUAI ET AL: "Treatment of surgically induced acute liver failure by transplantation of HNF4-overexpressing embryonic stem cells", CHINESE JOURNAL OF DIGESTIVE DISEASES, vol. 7, no. 2, 1 May 2006 (2006-05-01), pages 109-116, XP055067112, ISSN: 1443-9611, DOI: 10.1111/j.1443-9573.2006.00253.x
- IMAMURA T ET AL: "Embryonic stem cell-derived embryoid bodies in three-dimensional culture system form hepatocyte-like cells in vitro and in vivo", TISSUE ENGINEERING, LARCHMONT, NY, US, vol. 10, no. 11-12, 1 November 2004 (2004-11-01), pages 1716-1724, XP002555497, ISSN: 1076-3279, DOI: 10.1089/TEN.2004.10.1716
- KHURANA SATISH ET AL: "Hepatocyte nuclear factor-4alpha induces transdifferentiation of hematopoietic cells into hepatocytes", JOURNAL OF BIOLOGICAL CHEMISTRY, AMERICAN SOCIETY FOR BIOCHEMISTRY AND MOLECULAR BIOLOGY, INC, BETHESDA, MD, USA, vol. 285, no. 7, 12 February 2010 (2010-02-12), pages 4725-4731, XP009136894, ISSN: 1083-351X, DOI: 10.1074/JBC.M109.058198
- KUBO A. ET AL: 'The homeobox gene HEX regulates proliferation and differentiation of hemangioblasts and endothelial cells during ES cell differentiation' BLOOD vol. 105, no. 12, 22 February 2005, pages 4590 - 4597, XP008155481
- SHIMODA M. ET AL: 'Sox17 plays a substantial role in late-stage differentiation of the extraembryonic endoderm in vitro' J. CELL SCI. vol. 120, no. 21, 16 October 2007, pages 3859 - 3869, XP008155510
- CAI J. ET AL: 'Directed differentiation of human embryonic stem cells into functional hepatic cells' HEPATOLOGY vol. 45, no. 5, May 2007, pages 1229 - 1239, XP008155505
- SONG Z. ET AL: 'Efficient generation of hepatocyte-like cells from human induced pluripotent stem cells' CELL RES. vol. 19, no. 11, 08 September 2009, pages 1233 - 1242, XP008155513
- KUBO A. ET AL: 'The homeobox gene Hex regulates hepatocyte differentiation from embryonic stem cell-derived endoderm' HEPATOLOGY vol. 51, no. 2, 05 February 2010, pages 633 - 641, XP008155506
- INAMURA M. ET AL: 'Efficient Generation of Hepatoblasts From Human ES Cells and iPS Cells by Transient Overexpression of Homeobox Gene HEX' MOL. THER., [Online] 23 November 2010, XP008155514 Retrieved from the Internet: <URL:http://www.nature.com/mt/journal/vaop/ ncurrent/full/mt2010241a.html> [retrieved on 2011-01-12]
- CHEN M.L. ET AL: 'HNF-4a determines hepatic differentiation of human mesenchymal stem cells from bone marrow' WORLD J. GASTROENTEROL. vol. 16, no. 40, 28 October 2010, pages 5092 - 5103, XP008155517
- KAWABATA K. ET AL: 'Efficient gene transfer into mouse embryonic stem cells with adenovirus vectors' MOL. THER. vol. 12, no. 3, September 2005, pages 547 - 554, XP008155508
- NAIKI T. ET AL: 'Adenovirus-mediated hepatocyte nuclear factor-4alpha overexpression maintains liver phenotype in cultured rat hepatocytes' BIOCHEM. BIOPHYS. RES. COMMUN. vol. 335, no. 2, 23 September 2005, pages 496 - 500, XP008155516
- A P Li ET AL: "Applications of primary human hepatocytes in the evaluation of pharmacokinetic drug^drug interactions: Evaluation of model drugs terfenadine and rifampin Introduction", Cell Biology and Toxicology, 1 January 1997 (1997-01-01), pages 365-374, XP055250514, Retrieved from the Internet: URL:http://download.springer.com/static/pd f/959/art%3A10.1023%2FA%3A1007451911843.pd f?originUrl=http://link.springer.com/artic le/10.1023/A:1007451911843&token2=exp=1455 632484~acl=/static/pdf/959/art%253A10.1023 %252FA%253A1007451911843.pdf?originUrl=htt p%3A%2F%2Flink.springer.com%2Farticle%2F10 .1023%2FA%

## Description

### Technical Field

The present invention relates to a method of differentiation from pluripotent stem cells such as embryonic stem cells (hereinafter, also referred to as "ES cells") or induced pluripotent stem cells (hereinafter, also referred to as "iPS cells") to hepatocytes. The present invention also relates to stem cells containing an introduced gene that is useful for differentiation to hepatocytes.

This application claims the benefit of priority to Japan applications, JP-A Nos. 2009-247342, 2010-121282 and 2010-154225, which are incorporated herein by reference.

### Background Art

Pluripotent stem cells are undifferentiated cells that have pluripotency and self-replication competence and are suggested to have potency to repair damaged tissues. For that reason, pluripotent stem cells have been studied intensively, as they are considered useful in the field of screening for therapeutic drugs for various diseases and also in the field of regeneration medicine. Among the pluripotent stem cells, iPS cells (induced pluripotent stem cells) are stem cells prepared by dedifferentiation of somatic cells, such as fibroblasts, by introduction of a particular transcription factor, such as OCT3/4, SOX2, KLF4 or C-MYC, into the cells. Theoretically, totipotent cells can induce differentiation into all tissues and organs including liver.

Formation of spheroids (embryoid bodies), addition of a humoral factor to the medium, and addition for example of a suitable extracellular matrix, feeder cells and matrigel, as properly selected, were mainly studied as the methods for induction of differentiation from pluripotent stem cells to hepatocytes. However, it was reported that these methods demand longer culture period and are extremely lower in differentiation-inducing efficiency and that the hepatocytes thus obtained are also lower in drug-metabolizing enzyme activity (Nonpatent Literatures 1 to 4). For improvement of the differentiation-inducing efficiency, introduction of a gene playing an important role in differentiation may be introduced, for example, into pluripotent stem cells but, because there is no established method of introducing such a gene efficiently, there is almost no report on induction of differentiation into hepatocytes by gene introduction. For differentiation from stem cells to mature hepatocytes, the stem cells should differentiate via endodermal cells and hepatic stem cells into juvenile hepatocytes. In each differentiation step, a humoral factor, such as activin A, basic fibroblast growth factor (bFGF), BMP4 (bone morphogenetic protein 4), FGF4, retinoic acid or DMSO, is used in the culture system. In addition, transcription factors such as HEX, HNF4A, HNF6 and FOXA2 are reported to be required embryologically as the factors for inducing differentiation to hepatocytes (Nonpatent Literature 5). For example, HEX gene is found to be generated, for example, in thyroid gland, lung, liver, blood cells and vascular endothelial cells. HEX gene-deficient mice are found to have no hepatocyte and die approximately on the 10.5th day of viviparity (E10.5). The HEX gene is considered to regulate expression, for example, of GATA4, HNF4A and FGF receptor genes.

As described above, when FOXA2, a possible hepatocyte-specific transcription factor, was introduced into ES cells in development, there was observed differentiation from the ES cells to hepatocytes, but also to cells other than hepatocytes, as reported in literature (Nonpatent Literature 6). This fact indicates that FOXA2 is not a transcription factor that can induce differentiation of pluripotent stem cells such as ES cells only to hepatocytes, and the action of the factor is yet to be elucidated sufficiently.

A system employing an adenovirus (hereinafter, referred to simply as "Ad") was developed as a vector system for next-generation gene therapy. Ad has a regular icosahedral structure consisting of 252 capsomeres, 12 capsomeres thereof present on the vertices are called pentons, which has a protruded structure (consisting of penton base and fiber), and the other 240 capsomeres are called hexons. A virus enters cell, as the fiber binds to the Ad receptor (CAR; coxsackievirus-adenovirus receptor) and then the RGD motif of penton base binds to intgrins (αvβ3 and αvβ5) on the cell surface. However, in later studies various studies aimed at making the Ad vector incorporated into cells expressing no or little CAR were made and reported (Patent Documents 1 to 3). There is a report on gene delivery to mesenchymal stem cells by using such an improved Ad vector (Nonpatent Literature 7). It is disclosed here that genes are introduced for example into mesenchymal stem cells by using various improved Ad vectors. However, mesenchymal stem cells and ES cells are completely different from each other. In addition, the Nonpatent Literature only discloses that the Ad vector has a role of delivering a gene into cells (Drug Delivery System). It was reported that Ad vectors can be used as a tool for differentiation of mouse ES and iPS cells (Nonpatent Literatures 8 and 9). However, these Nonpatent Literatures do not disclose a method of inducing differentiation from stem cells to hepatocytes.

### Prior Art literature

### Patent Documents

Patent Document 1: JP-ANo. 2002-272480 (Patent 3635462)
Patent Document 2: JP-ANo. 2003-250566
Patent Document 3: JP-ANo. 2008-136381

### Nonpatent Literatures

Nonpatent Literatures 1: Genes to Cells, 13, 731-746 (2008)
Nonpatent Literatures 2: Stem Cells, 26, 894-902 (2008)
Nonpatent Literatures 3: Stem Cells, 26, 1117-1127 (2008)
Nonpatent Literatures 4: Hepatology, 45, 1229-1239 (2007)
Nonpatent Literatures 5: Nature Reviews Genetics, 3, 499-215 (2002)
Nonpatent Literatures 6: FASEB Journal, 16, 1444-1446 (2002)
Nonpatent Literatures 7: Biochem. Biophys. Res. Commun., 332, 1101-1106 (2005)
Nonpatent Literatures 8: Mol Ther., 12 (3), 547-554 (2005)
Nonpatent Literatures 9: Stem Cells, 27 (8), 1802-1811 (2009)

### Summary of the Invention

### Problems to be Solved by the Invention

An object of the present invention is to provide a method of differentiation from stem cells to hepatocytes by introducing a gene involved in differentiation into pluripotent stem cells such as ES or iPS cells. Another object of the present invention is to provide a stem cell containing a gene useful for induction of differentiation into tissue cells and a hepatocyte generated by differentiation from the stem cells containing the gene introduced.

### Means to Solve the Problems

After intensive studies to achieve the objects above, the inventors have found that it is possible to introduce a particular gene into pluripotent stem cells such as ES or iPS cells by using an Ad vector and to induce differentiation to hepatocytes effectively by introducing a particular gene using the vector, and made the present invention.

Specifically, the present invention includes the following aspects:
1. a method of differentiation from stem cells to hepatocytes, characterized by introducing a gene into stem cells using an Ad vector;
2. the method of differentiation described in aspect 1, wherein the gene introduced is one or more genes selected from HEX, HNF4A, HNF6 and SOX17 genes;
3. the method of differentiation described in aspect 1 or 2, wherein at least the HEX gene is introduced and additionally the HNF4A gene and/or the SOX17 gene are introduced;
4. the method of differentiation described in aspect 3, wherein the SOX17 gene and then the HEX gene are introduced into stem cells.
5. the method of differentiation described in aspect 3 or 4, wherein the HEX gene and then the HNF4A gene are introduced into stem cells;
6. a method of differentiation from stem cells to hepatocytes, comprising the steps of:
   1) introducing an HEX gene cassette into an Ad vector;
   2) introducing the HEX gene into cells by bringing the Ad vector containing the gene introduced in step 1) with stem cells; and
   3) culturing the stem cells containing the gene introduced therein;
7. the method of differentiation from stem cells to hepatocytes described in aspect 6, comprising, before the steps 1) to 3) above, the following steps of:
   a) introducing an SOX17 gene cassette into an Ad vector;
   b) introducing the SOX17 gene into cells by bringing the Ad vector containing the gene introduced in step a) with stem cells; and
   c) culturing the stem cells containing the gene introduced therein;
8. the method of differentiation from stem cells to hepatocytes described in aspect 6 or 7, comprising, after the step of culturing the stem cells containing the HEX gene introduced in step 3), a step of introducing an HNF4A gene into the cells additionally by bringing the cultured cell into contact with an Ad vector containing the HNF4A gene introduced therein;
9. the method of differentiation described in any one of aspects 1 or 8, wherein the stem cells are treated with a humoral differentiation-inducing factor;
10. the method of differentiation described in any one of aspects 1 or 9, wherein the stem cell is an embryonic stem cell or an induced pluripotent stem cell;
11, the differentiation-inducing method described in aspect 10, wherein the embryonic stem cell or the induced pluripotent stem cell is a human embryonic stem cell or a human induced pluripotent stem cell;
12. a stem cell containing a gene introduced by the method of differentiation described in to any one of aspects 1 to 11;
13. a hepatocyte differentiated from the stem cell containing an introduced gene described in aspect 12; and
14. a method of using the hepatocyte described in aspect 13 for evaluation of pharmaceutical toxicity or pharmacokinetics.

### Advantageous Effect of the Invention

It was found that it is possible, by using the Ad vector according to the present invention, to introduce a gene involved in induction of differentiation of pluripotent stem cells such as ES or iPS cells effectively and to induce differentiation to hepatocytes. Specifically, it is possible to induce differentiation from stem cells such as ES or iPS cells to hepatocytes effectively by introducing one or more genes selected from HEX, HNF4A, HNF6 and SOX17 genes into the stem cells. In particular, it was confirmed that it is possible to induce differentiation from stem cells to hepatocytes effectively by introducing HEX gene into stem cells. It was further confirmed that it is possible to induce differentiation from stem cells to hepatocytes more effectively by introducing additionally other genes such as SOX17 gene and/or HNF4A gene, as needed, according to the progress of cell differentiation.

### Brief Description of the Drawings

FIG. 1 is a chart showing the scheme of the test on induction of differentiation from iPS cells to hepatocytes by introducing an HEX gene into human iPS cells with an Ad vector. (Examples 1 and 3)
FIG. 2 is photographs by immunostaining, showing progress of differentiation from human iPS cells to hepatocytes. (Test Example 1-1)
FIG. 3 is a graph showing the results obtained when the progress of differentiation to hepatocytes when human iPS cells were cultured under various culture conditions was monitored, as compared to expression of α-fetoprotein (AFP) as indicator. (Test Example 1-2)
FIG. 4 is a graph showing the results obtained when the progress of differentiation to hepatocytes when human iPS cells were cultured under various culture conditions was monitored, as compared to expression of albumin as indicator. (Test Example 1-2)
FIG. 5 is a graph showing the results obtained when the progress of differentiation to hepatocytes when human ES cells were cultured under various culture conditions was monitored, as compared to expression of AFP as indicator. (Test Example 2)
FIG. 6 is a graph showing the results obtained when the progress of differentiation to hepatocytes when human ES cells were cultured under various culture conditions was monitored, as compared to expression of albumin as indicator. (Test Example 2)
FIG. 7 is photographs by immunostaining, showing progress of differentiation from human iPS cells to hepatocytes. (Test Example 3-1)
FIG. 8 is a chart showing the scheme of the test on induction of differentiation from iPS cells to hepatocytes by introducing an HEX gene into human iPS cells with an Ad vector. (Example 4)
FIG. 9 is a graph showing the results obtained when the progress of differentiation to hepatocytes when human iPS cells were cultured under various culture conditions was monitored, as compared to expression of AFP and albumin as indicators. (Test Example 4-1)
FIG. 10 is photographs by immunostaining, showing progress of differentiation from human iPS cells to hepatocytes. (Test Example 4-2)
FIG. 11 is a chart showing the scheme of the test on induction of differentiation from iPS cells to hepatocytes by introducing an HEX gene into human iPS cells with an Ad vector. (Example 5)
FIG. 12 is graphs showing the results obtained when the expression amounts of endoderm markers FOXA2 and SOX17 when 3 kinds of human stem cells were cultured were monitored. (Test Example 5-1)
FIG. 13 is graphs showing the results obtained when progress of differentiation to hepatocytes when 3 kinds of human stem cells were cultured was monitored, as compared to expression of α-fetoprotein (AFP) and albumin as indicators. (Test Example 5-1)
FIG. 14 is a chart showing the scheme of the test on induction of differentiation from iPS cells to hepatocytes by introducing an HEX gene into human iPS cells with an Ad vector. (Example 6)
FIG. 15 is photographs by immunostaining, showing progress of differentiation from human iPS cells to hepatocytes. (Test Example 6-1)
FIG. 16 is a chart showing the scheme of the test on induction of differentiation from iPS cells to hepatocytes by introducing an HEX gene into human iPS cells with an Ad vector. (Examples 7 and 8)
FIG. 17 is a graph showing the results obtained when the function of the HEX gene-introduced human iPS cells (Tic cell line) was analyzed by expression of drug-metabolizing enzyme cytochrome P450 3A4 (GYP3A4). (Test Example 7-1)
FIG. 18 is a graph showing the results obtained when the function of the HEX gene-introduced human iPS cells was analyzed by function of drug-metabolizing enzyme cytochrome P450 3A4 (CYP3A4) and drug response. (Test Example 7-2)
FIG. 19 is graphs showing the results when progress of differentiation from HEX gene-introduced human iPS cells (Tic cell line and Dotcom cell line) to hepatocytes was analyzed as compared to expression of albumin as indicator and function of the cells analyzed by expression of drug-metabolizing enzyme cytochrome P450 3A4 (CYP3A4). (Test Example 8-1)
FIG. 20 is a chart showing the scheme of the test on induction of differentiation from iPS cells to hepatocytes by introducing an HEX gene into human iPS cells with an Ad vector. (Example 9)
FIG. 21 is photographs showing the morphological change of the HEX gene-introduced human iPS cells (Tic cell line). (Test Example 9-1)
FIG.22 is a chart showing the scheme of the test on induction of differentiation from iPS cells to hepatocytes by introducing SOX17 and HEX genes into human iPS cells with an Ad vector. (Example 10)
FIG. 23 is photographs by immunostaining, showing progress of differentiation from human iPS cells to hepatocytes. (Test Example 10-1)
FIG. 24 is graphs showing the results obtained when progress of differentiation to hepatocytes, when human iPS cells were cultured under various conditions, was monitored, as compared with expression of AFP and albumin as indicators. (Test Example 10-2)
FIG. 25 is a chart showing the scheme of the test on induction of differentiation from iPS cells to hepatocytes by introducing HEX and HNF4A genes into human iPS cells with an Ad vector. (Example 11)
FIG. 26 is photographs by immunostaining, showing expression of drug-metabolizing enzyme cytochrome P450 3A4 (CYP3A4) in human iPS cells containing each gene introduced thereto. (Test Example 11-1)
FIG. 27 is graphs showing the results when the function of the HEX and HNF4A gene-introduced human iPS cells was analyzed, as compared to expression of drug-metabolizing enzyme cytochromes P450 2D6 (CYP2D6), 3A4 (CYP3A4), and 7A1 (CYP7A1). (Test Example 11-2)
FIG. 28 is a graph showing the results when the function of the HEX and HNF4A gene-introduced human iPS cells was analyzed by drug-metabolizing activity. (Test Example 11-3)
FIG. 29 is a chart showing the scheme of the test on induction of differentiation from iPS cells to hepatocytes scheme by introducing SOX17, HEX and HNF4A genes into human iPS cells with an Ad vector. (Example 12)
FIG. 30 is graphs showing the results when the function of the human iPS cells containing one or more genes selected from SOX17, HEX and HNF4A genes was analyzed, as compared to expression of drug-metabolizing enzyme cytochromes P450 2D6 (CYP2D6), 3A4 (CYP3A4) and 7A1 (CYP7A1).(Test Example 12)

### Favorable Embodiments of the Invention

The present invention relates to a method of introducing a gene into stem cells by using an Ad vector and thus inducing differentiation from stem cells to hepatocytes. In the present invention, the stem cell is a pluripotent stem cell such as ES cell or iPS cell, particularly favorably a human ES cell or a human iPS cell, but cells that can differentiate into the directed hepatocyte by introduction of a gene into human ES or iPS cells, such as mesendodermal, endodermal and hepatic stem cells, are also included. Examples of the hepatocytes according to the present invention, more specifically hepatocytes differentiated from the stem cells containing an introduced gene, include mature hepatocytes and also cells can differentiate into directed hepatocytes by introduction of a gene into stem cells, such as hepatic stem cells and juvenile hepatocytes.

Alternatively, the iPS cell is an induced pluripotent stem cell that is pluripotent and proliferating similarly to ES cells that was prepared without use of eggs, embryos or ES cells by reprogramming differentiated cell by introduction of several kinds of genes into a somatic cell and was established from a mouse fibroblast in 2006 for the first time in the world. It was also reported that human iPS cells were successfully established with four human genes OCT3/4, SOX2, KLF4 and C-MYC homologous to the four genes used in establishing mouse iPS cells (Cell 131: 861-872, 2007) introduced in a human fibroblast. The iPS cell for use in the present invention may be an iPS cell prepared by such a known method or an iPS cell that is prepared by a new method developed in the future.

The method of culturing the stem cells such as ES or iPS cells is not particularly limited and may be any known method. A known medium or a new medium to be developed in the future may be used as the medium for keeping the ES cells in the undifferentiated and pluripotent state and also as the medium suitable for induction of differentiation. Typical examples thereof include commercially available basal media for mammal cells, such as DMEM and/or DMEM/F12, containing serum or knock-out serum replacement (KSR) and bFGF and others added thereto, commercially available media for primate ES cells, a basal medium hESF-GRO for proliferation of primate ES cells, a basal medium hESF-DIF for induction of differentiation of primate ES cells, and a medium CSTI-7 for proliferation of primate ES cells. The medium may contain known additives suitable for use in culture of pluripotent stem cells such as ES or iPS cells, for example one or more additives selected from N2 supplement, B27 supplement, insulin, bFGF, activin A, heparin, various inhibitors such as ROCK inhibitor and GSK-3 inhibitor and others, at suitable concentrations. The media and the additives can be used, as they are selected properly for example according to the cells used and the differentiation stage. For example, they may be selected by the method described in Tiss, Cult. Res. Commun., 27: 139-147 (2008).

The Ad vector used in the present invention is not particularly limited and an Ad vector prepared by a known method may be used. For example, it may be an improved Ad vector improved for introduction thereof into cells expressing no or very limited CAR or an Ad vector for introduction thereof into cells expressing CAR. Specifically, it is possible to use Ad vectors containing a DNA coding an cell adhesion peptide (RGD sequence), a representative of adhesion peptides, a DNA coding a peptide compatible with heparan sulfate (K7 (KKKKKKK) sequence), a DNA coding a peptide compatible with laminin receptor, or a DNA coding a peptide compatible with E-selectin, and also the Ad vectors for example shown in Patent Documents 1 to 3.

A desired gene shown below can be introduced into the Ad vector by any known method or any method to be developed in the future. Such a gene can be introduced into the Ad vector according to the present invention by cleaving the vector with one or more restriction enzymes at the respective recognition sequences, introducing the gene there with or without a shuttle vector and ligating the product in vitro.

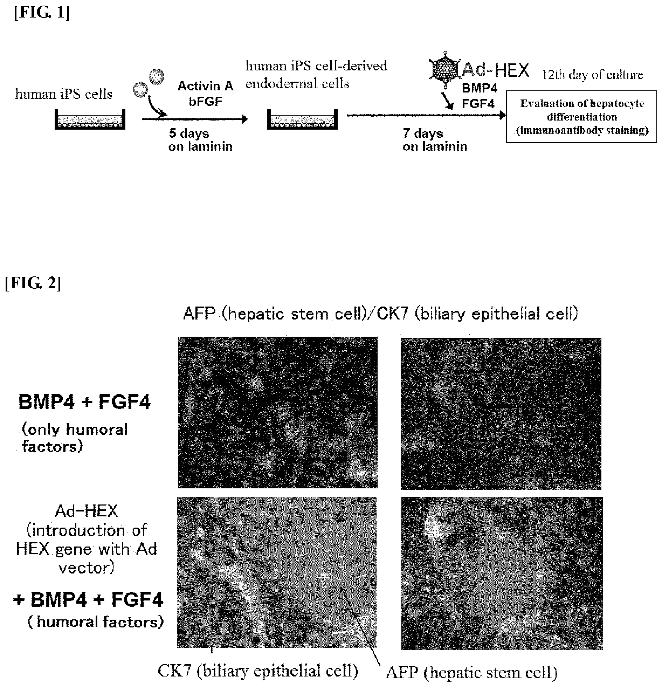

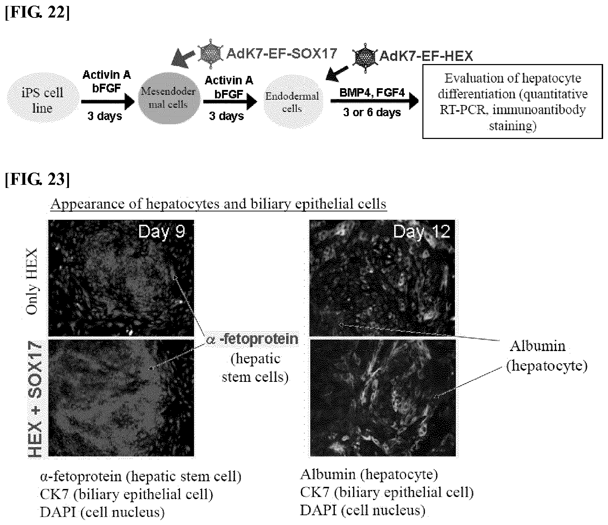

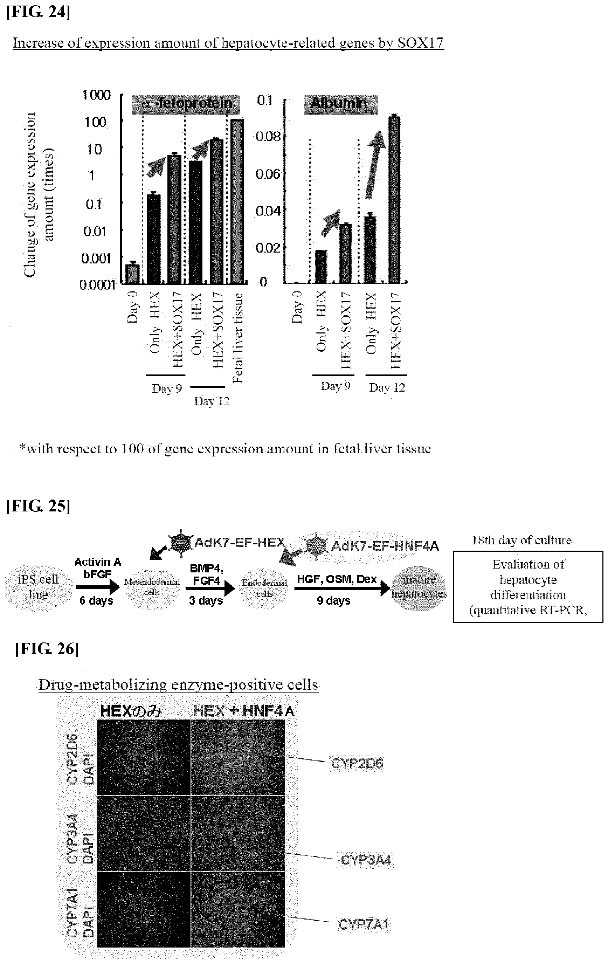

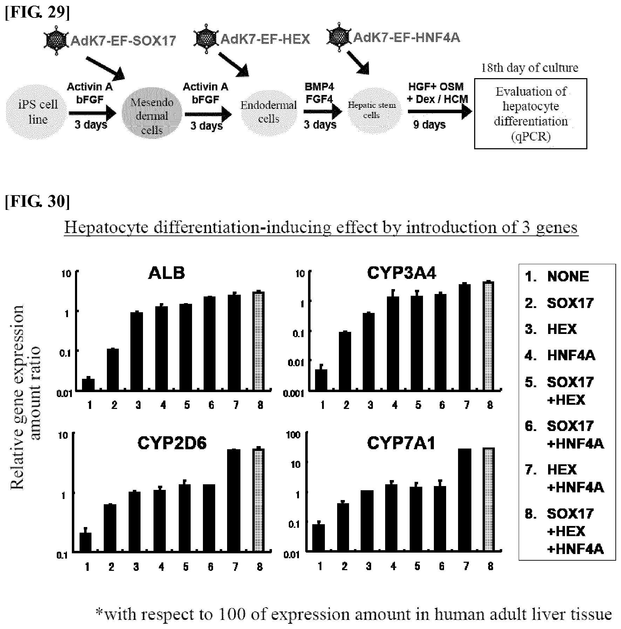

In the present invention, the gene to be introduced by using the Ad vector may be any gene that can induce differentiation of pluripotent stem cells such as ES or iPS cells to hepatocytes effectively. Specifically, it is possible to introduce a gene selected from HEX, HNF4A, HNF6 and SOX17 genes into stem cells, and in particular, introduction of HEX gene is favorable (see FIGS. 1, 8, 11, 14, 16 and 20). Introduction of the HEX gene results in induction of differentiation from stem cells to hepatocytes. For effective induction of differentiation to hepatocytes, it is favorable to introduce the SOX17 gene first, determining the directing of differentiation from stem cells to endodermal cells, and introduce the HEX gene then (see FIG. 22). It is also possible to determine the directing of differentiation to hepatocytes by introducing the HEX gene and then promote differentiation to hepatocytes by introducing a gene selected from HNF4A gene, HNF6 gene and FOXA2 gene, particularly preferably HNF4A gene (see FIG. 25). For the most effective induction of differentiation from stem cells to hepatocytes, it is considered most preferable to introduce the SOX17 gene, thus determining the directing of differentiation from stem cells to hepatocytes, and the HEX gene and additionally the HNF4A gene (see FIG. 29). For example, it is possible to induce differentiation from ES or iPS cells to hepatocytes by the operations in the following Steps 1) to 3):
1) a step of introducing a gene that can effectively induce differentiation from stem cells such as ES or iPS cells to hepatocytes into an Ad vector,
2) a step of bringing the Ad vector having the gene introduced therein prepared in step 1) above in contact with stem cells and thus introducing a gene that can effectively induce differentiation from stem cells to hepatocytes into the stem cells, and
3) a step of culturing the gene-containing stem cells.

In the step 1), the gene that can effectively induce differentiation from stem cells to hepatocytes is a gene selected from HEX, HNF4A and HNF6 genes, more preferably HEX gene. It is possible to direct differentiation to hepatocytes more effectively by introducing the SOX17 gene into the stem cells before introduction of the gene. Further, it is possible to induce differentiation to hepatocytes more effectively, by introducing a gene selected from HNF4A, HNF6 and FOXA2 genes, particularly favorably HNF4A gene into the cultured cells after step 3). It would be possible to prepare hepatocytes from iPS cells efficiently, by introducing, in addition to SOX17, HEX and HNF4A, one or more genes that are involved in differentiation and proliferation of hepatocytes, such as GATA4, GATA6, HNF1A, HNF1B, FOXA1/HNF3A, FOXA2/HNF3B, FOXA3/HNF3G, CEBPA, CEBPB, TBX3 and PROX1. The step of introducing each gene can be selected properly according to the differentiation stage of the cells. For preparation of biliary epithelial cells surrounding hepatocytes, it would be effective to introduce Sall4 and HNF6 genes. These genes are introduced into the stem cells, by the Ad vectors containing desired genes into contact with the stem cells according to the differentiation stage of the cells.

As for the genes to be introduced, the HEX gene used may be, for example, the gene of GenBank Accession No. BC014336; the HNF4A gene used, the gene of GenBank Accession No. NM000457; the HNF6 gene used, the gene of GenBank Accession No. NM004498; the FOXA2 gene used, the gene of GenBank Accession No. BC011780; and the SOX17 gene used, the gene of GenBank Accession No. NM_022454.

The Ad vector according to the present invention can be prepared by a method comprising the following steps A) and B):
A) a step of constructing an expression construct containing a promoter sequence in the noncoding region of the introduced gene, and
B) a step of cleaving the Ad genome with a restriction enzyme and ligating the expression construct prepared in step A) into the Ad genome.

The Ad vector according to the present invention may be prepared by constructing a shuttle vector containing the expression construct of step A) and ligating the gene expression shuttle vector into the Ad genome between the steps A) and B) above.

It is known in the art that humoral factors such as activin A, bFGF, BMP4 and FGF-4 are used in the differentiation steps from undifferentiated cells to mature hepatocytes. The humoral factors may be brought into contact with the cells in combination also in the step of induction of differentiation from stem cells such as ES or iPS cells to hepatocytes according to the present invention. The step of bringing the humoral factors into contact with the cells is not particularly limited and may be carried out before or after introduction of the various selected genes above. If multiple genes are to be introduced, the humoral factors may be brought into contact with the cells between the introduction steps of respective genes. Adhesion between cells or between cells and other cells or substrate is needed for growth and division of normal cells, and proteins (extracellular matrix) present between cells or between the cells and the substrate are also needed. In the method of inducing differentiation to hepatocytes according to the present invention, the extracellular matrices described above may be added in the steps above. Examples of the extracellular matrices include matrigel, fibronectin, vitronectin, laminin, nidogen, tenascin, thrombospondin, type-I collagen, type-IV collagen, gelatin, and synthetic substrates equivalent thereto, and in the present invention, matrigel or laminin can be used particularly favorably. Specifically, they can be used, as they are coated on or added to a container for culture.

The present invention also relates to a stem cell such as ES or iPS cell containing an Ad vector having the gene introduced therein. It also relates to a hepatocytes generated from the stem cell containing the Ad vector having the gene introduced therein.

It is possible by using hepatocytes prepared by the method of inducing differentiation according to the present invention to examine pharmaceutical toxicity and pharmacokinetics. Specifically, it is possible to predict the toxicity of a candidate drug compound in the body by adding the candidate drug compound to the hepatocytes prepared according to the present invention and analyzing expression and fluctuation of hepatotoxicity markers. It is also possible to predict the pharmacokinetics (metabolite) of a candidate drug compound in the body by adding the candidate drug compound to the hepatocytes prepared according to the present invention and analyzing the metabolites of the compound. It is possible in this way to screen candidate drug compounds to be eliminated because of problems in toxicity and pharmacokinetics rapidly and thus to accelerate drug development. The present invention also relates to a method of using the hepatocytes prepared by the method of inducing differentiation according to the present invention for evaluation of pharmaceutical toxicity and pharmacokinetics.

### Examples

Hereinafter, the present invention will be described more specifically with reference to Examples and test Examples for deeper understanding of the present invention, but it should be understood that the scope of the present invention is not restricted by these examples.

Various media are needed for human iPS or ES cells in Examples and Test Examples. Compositions of the various media used will be described in Reference Example below and the differentiation-inducing methods then in the Examples below.

### (Reference Example) composition of various media

1) A medium in the composition shown in Table 2 of Tiss. Cult. Res. Commun., 27: 139-147 (2008), wherein the basic medium is knock-out DMEM/F12 and which contains bFGF (10 ng/ml), was used as the medium for keeping human iPS cells undifferentiated. Hereinafter, the medium will be referred to as "medium 1."
2) A medium in the composition shown in Table 2 of Tiss. Cult. Res. Commun., 27: 139-147 (2008), wherein basic medium is DMEM/F12 and which contains bFGF (5 ng/ml), was used as the medium for keeping human ES cells undifferentiated. Hereinafter, the medium will be referred to as "medium 2."
3) A basic medium for culturing human ES cells, i.e., hESF-GRO medium (Cell Science & Technology Institute, Inc.), containing insulin (10 µg/ml), transferrin (5 µg/ml), albumin conjugate oleic acid (9.4 µg/ml), 2-mercaptoethanol (10 µM), 2-ethanolamine (10 µM), sodium selenite (20 nM), heparin (100 ng/ml) and bFGF (10 ng/ml) (Proc. Natl. Acad. Sci. USA; 105 (36): 13409-13414 (2008)) was used as the medium for keeping cells undifferentiated. Hereinafter, the medium will be referred to as "medium 3." The medium 3, which demands no feeder cell or Knock-out Serum Replacement (KSR), permits culture of human iPS cells, as the cells are kept pluripotent in the undifferentiated state.
4) A basic medium for culturing human ES cells, i.e., hESF-GRO (Cell Science & Technology Institute, Inc.) containing insulin (10 µg/ml), transferrin (5 µg/ml), 2-mercaptoethanol (10 µM), 2-ethanolamine (10 µM), sodium selenite (20 nM) and bovine serum albumin (BSA) (1 mg/ml) was used as the medium for inducing differentiation. Hereinafter, the medium will be referred to as "medium 4."
5) A basic medium for inducing differentiation of human ES cells, i.e., hESF-DIF (Cell Science & Technology Institute, Inc.) containing insulin (10 µg/ml), transferrin (5 µg/ml), 2-mercaptoethanol (10 µM), 2-ethanolamine (10 µM), sodium selenite (20 nM) (FASEB J. 23: 114-22 (2009)) was used as another embodiment of the medium for inducing differentiation. Hereinafter, the medium will be referred to as "medium 5."

### (Example 1) Method of inducing differentiation from human iPS cells to hepatocytes

In this Example, induction of differentiation from human iPS cells containing the HEX gene introduced by using Ad vector to hepatocytes will be described. The experimental scheme in this Example is shown in FIG. 1.

### 1) Construction of Ad vector for introduction of HEX gene

An Ad vector having a gene introduced into the E1-deficient region of a E1-deficient type-5 Ad genome (pAdHM41-K7) and an EF-1α promoter in the region upstream thereof was prepared. The gene introduced was the HEX gene having the sequence of GenBank Accession No. BG014336.

### 2) Culture of human iPS cells

Human iPS cells (Tic (JCRB1331), Dotcom (JCRB1327), and Squeaky (JCRB1329)) were used in this Example. Cultured human iPS cells were obtained by using mouse fetal fibroblasts (MEF) as feeder cells and the medium 1 shown in Reference Example according to the method described in Tiss. Cult. Res. Commun., 27:139-147 (2008). The culture medium was replaced with the medium 3 shown in Reference Example, two days before induction of differentiation of the human iPS cells.

The culture solution was then treated with a cell dissociation solution Accutase® (Invitrogen) at 37°C for 3 minutes and the supernatant containing floating feeder cells was removed. Human iPS cells were then collected by using the medium 3 and the cell suspension was centrifuged at 1,300 rpm for 3 minutes. The cells were resuspended in the medium 4 shown in Reference Example and centrifuged at 1,300 rpm for 3 minutes additionally twice.

The human iPS cells obtained by centrifugation were resuspended in the medium 4 containing 50 ng/ml of activin A (R&D Systems) and 10 ng/ml of bFGF (R&D Systems); the human iPS cells were inoculated in each well of a laminin (sigma)-coated cell culture plate (12 wells) in an amount of 2.5×10⁵ cell/well; the medium was replaced every day with the medium 4 containing 50 ng/ml of activin A (R&D Systems) and 10 ng/ml of bFGF (R&D Systems) cultured at 37°C. The day when the human iPS cells were inoculated is the 0th day of differentiation induction.

### 3) Introduction of HEX gene into human iPS cells

On the 5th day from differentiation induction, the human iPS cells were separated from the cell culture plate with 0.0125% trypsin-0.01325 mM EDTA; and after trypsin was neutralized with 0.1 mg/ml trypsin inhibitor, the cells were centrifuged from the medium 5 shown in Reference Example twice at 1,300 rpm for 3 minutes. The human iPS cells obtained after centrifugation was resuspended in the medium 5 containing 50 ng/ml of activin A (R&D Systems) and 10 ng/ml of bFGF (R&D Systems), inoculated in each well of a laminin (sigma)-coated cell culture plate (12 wells) in an amount of 5.0×10⁵ cell/well and cultured at 37°C.

After 24 hours, the cells were infected with the AdK7 (Ad vector containing K7 peptide in the fiber knob region) vector containing the HEX gene introduced in step 1) at a concentration of 3,000 VP (vector particle)/cell for 1.5 hours, and the medium was replaced with the medium 5 containing 10 ng/ml of BMP4 (R&D Systems) and 10 ng/ml of FGF-4 (R&D Systems) added thereto. The medium was then replaced every day with the medium 5 containing 10 ng/ml of MBP4 (R&D Systems) and 10 ng/ml of FGF-4 (R&D Systems) added thereto.

### (Test Example 1-1) Results obtained by immunostaining method

On the 12th day of culture, expression of each gene was examined by an immunostaining method using Alexa®594-labeled anti-α-fetoprotein (AFP) antibody (manufactured by Dako) and Alexa®488-labeled anti-CK7 antibody (manufactured by Invitrogen). AFP is a marker for hepatic stem cells and CK7, a marker for biliary epithelial cells. As shown in FIG. 2, it was found that both markers reacted significantly in the systems containing the HEX gene introduced with the Ad vector than in the systems without the gene introduced. The results show that introduction of HEX gene using the Ad vector leads to enhanced induction of differentiation from human iPS cells to hepatocytes.

### (Test Example 1-2) Results obtained by real-time PCR method

The expression amounts of possible hepatic stem cell markers AFP and albumin were determined on the 0th day of culture (undifferentiated cell), 5th day (endodermal cell) and 12th day by a real-time PCR method of using TaqMan® Gene Expression Assays (Applied Biosystems, catalog number: Hs01040607_1 for AFP and Hs00910225 _m for albumin). The expression amounts were calculated, based on the standard (100) of the expression amount of each gene in Human Fetal Liver Total RNA (Clontech, catalog number: 636540).

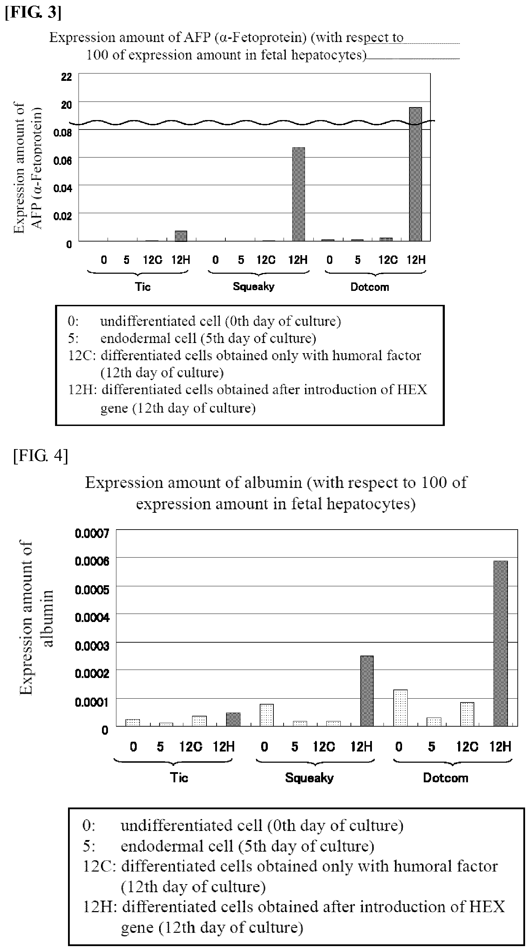

The results showed definitely that both AFP and albumin were expressed in greater amounts in the cells overexpressing the HEX gene and thus, differentiation was induced more intensively in the HEX gene-overexpressing cells (FIGS. 3 and 4).

### (Example 2) Method of differentiation from human ES cells to hepatocytes

In this Example, HEX gene was introduced by using an Ad vector into the human ES cells (KhES-1) established by the Inst. for Frontier Medical Science, Kyoto Univ., replacing the human iPS cells above, and induction of differentiation from human ES cells to hepatocytes was studied. In this Example, 1) an Ad vector for introduction of HEX gene was constructed, 3) the HEX gene was introduced into human ES cells, and the test was performed in a manner similar to Example 1, except that the cultured human ES cells were prepared by using mouse fetal fibroblasts (MEF) as the feeder cells and the medium 2 shown in Reference Example according to the method described in Tiss. Cult, Res. Commun., 27: 139-147 (2008).

### (Test Example 2) Results obtained by real-time PCR method

The expression amounts of AFP and albumin, indicators of differentiation, were determined by a real-time PCR method on the 0th day of culture (undifferentiated cell), 5th day (endodermal cell) and 12th day by the method similar to that in Example 1-2

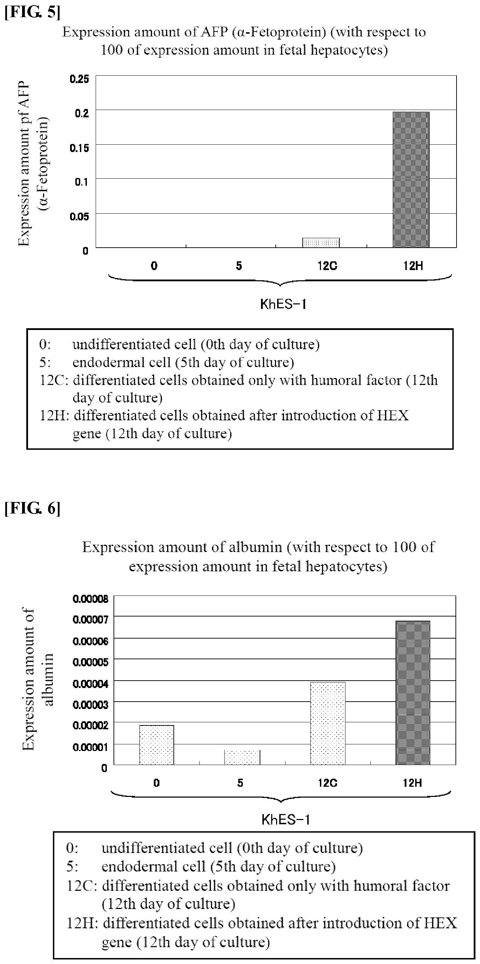

The results showed definitely that both AFP and albumin were expressed in greater amounts in the system containing the HEX gene and thus, differentiation was induced significantly in HEX gene-containing systems (FIGS. 5 and 6).

### (Example 3) Method of differentiation from human iPS cells to hepatocytes

In this Example, induction of differentiation from human iPS cells containing the HEX gene introduced by using an Ad vector to hepatocytes will be described. The experimental scheme in this Example is shown in FIG. 1. 1) Construction of an Ad vector for introduction of HEX gene, 2) culture of human iPS cells and 3) introduction of HEX gene into human iPS cells were carried out in a manner similar to Example 1. Human iPS cells (Squeaky (JCRB1329)) were used.

### (Test Example 3-1) Results obtained by immunostaining method

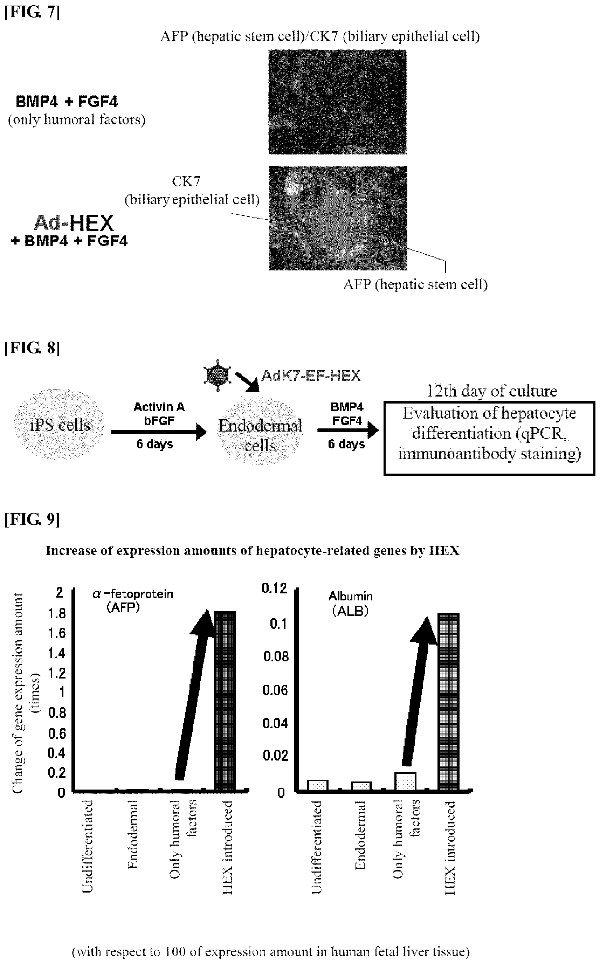

On the 12th day of culture, expression of each gene of AFP and CK7 was examined by an immunostaining method using Alexa®594-labeled anti-AFP antibody (manufactured by Dako) and Alexa®488-labeled anti-CK7 antibody (manufactured by Invitrogen). AFP is a marker for hepatic stem cells and CK7 a marker for biliary epithelial cells. As shown in FIG. 7, it was found that both markers reacted significantly in the systems containing the HEX gene introduced with the Ad vector than in the systems without the gene introduced. The results show that introduction of HEX gene using the Ad vector leads to enhanced induction of differentiation from human iPS cells to hepatocytes.

### (Example 4) Method of differentiation from human iPS cells to hepatocytes

In this Example, induction of differentiation from human iPS cells containing the HEX gene introduced by using an Ad vector to hepatocytes will be described. The experimental scheme in this Example is shown in FIG. 8. 1) Construction of an Ad vector for introduction of HEX gene, 2) culture of human iPS cells and 3) introduction of HEX gene into human iPS cells were carried out in a manner similar to Example 1. Human iPS cells (Tic (JCRB1331)) were used.

### (Test Example 4-1) Results obtained by real-time PCR method

The expression amounts of possible hepatic stem cell markers AFP and albumin were determined on the 0th day of culture (undifferentiated cell), 5th day (endodermal cell) and 12th day by a real-time PCR method of using TaqMan® Gene Expression Assays (Applied Biosystems, catalog number: Hs01040607_1 for AFP and Hs00910225 _m for albumin). The expression amounts were calculated, based on the standard (100) of the expression amount of each gene in Human Fetal Liver Total RNA (Clontech, catalog number: 636540).

The results showed definitely that both AFP and albumin were expressed in greater amounts in the cells overexpressing the HEX gene and thus, differentiation was induced more intensively in HEX gene- overexpressing cells (FIG. 9).

### (Test Example 4-2) Results obtained by immunostaining method

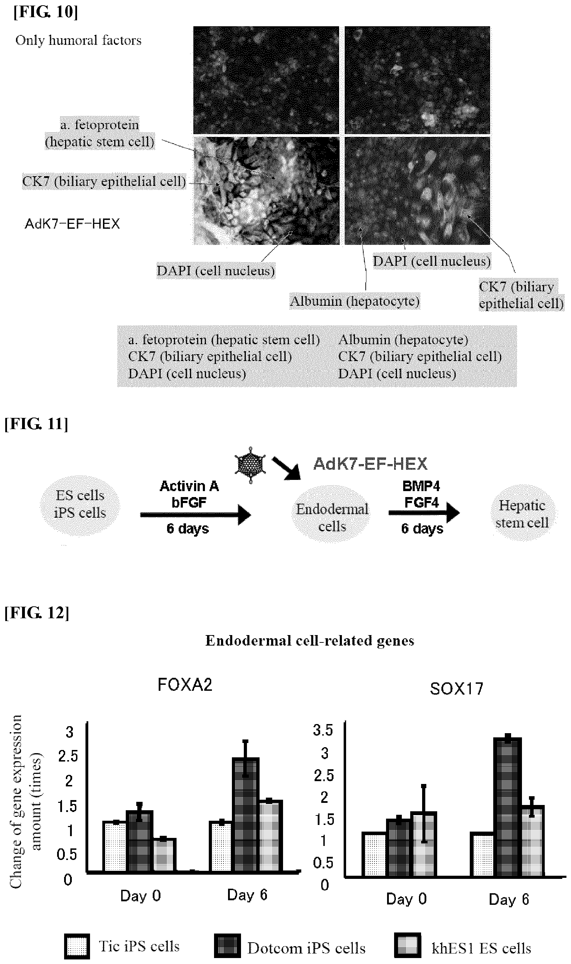

On the 12th day of culture, expression of genes of AFP, albumin and CK7 was determined by an immunostaining method, by using Alexa®594-labeled anti-AFP antibody (manufactured by Dako), Alexa®594-labeled anti-albumin (ALB) antibody (manufactured by SIGMA) and Alexa®488-labeled anti-CK7 antibody (manufactured by Invitrogen). AFP is the marker for hepatic stem cells, albumin, the marker for hepatocytes, and CK7, the marker for biliary epithelial cells. As shown in FIG. 10, it was found that these markers reacted significantly in the cells overexpressing the HEX gene introduced with the Ad vector than in the cells without the gene introduced. The results show that introduction of HEX gene using the Ad vector leads to enhanced induction of differentiation from human iPS cells to hepatocytes.

### (Example 5) Method of induction of differentiation from human iPS and ES cells to hepatocytes

### 1) Culture of human iPS and ES cells

In this Example, induction of differentiation from gene-containing human iPS and ES cells, which are prepared by introducing the HEX gene into human iPS and ES cells using an Ad vector, to hepatocytes will be described. The experimental scheme in this Example is shown in FIG. 11. 1) Construction of an Ad vector for introduction of HEX gene, 2) culture of human iPS cells and 3) introduction of HEX gene into human iPS cells were carried out in a manner similar to Example 1. The human iPS cells used were Tic (JCRB1331) and Dotcom (JCRB1327) and the ES cells used were khES1 ES cells.

### (Test Example 5-1) Results obtained by real-time PCR method

The expression amounts of the genes of possible endodermal cell markers FOXA2 and SOX17 and possible hepatic stem cell markers AFP and albumin in the cells on the 0th day of culture (undifferentiated cells), 6th day (endodermal cells) and 12th day were determined. Expression of respective genes was determined by a real-time PCR method of using TaqMan® Gene Expression Assays (Applied Biosystems, catalog number: Hs00232764_m1 for FOXA2, Hs00751752_s1 for SOX17, Hs01040607_m1 for AFP and Hs00910225_m for albumin). The expression amounts were calculated, based on the standard (1) of the cells of human iPS cell Tic on the 0th day of culture (undifferentiated cells).

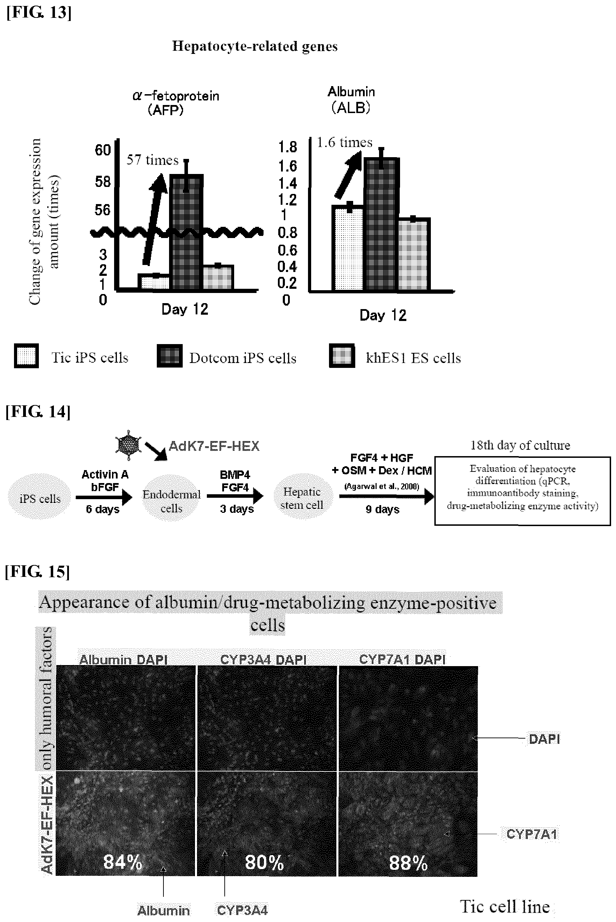

As shown in FIG. 12, the expression amounts of the endodermal cell markers FOXA2 and SOX17 were the largest in the cell line Dotcom on the 6th day (endodermal cells). As shown in FIG. 13, the possible hepatic stem cell marker AFP and the possible hepatocyte marker albumin showed the largest increase in expression amount in the cell line Dotcom when the HEX gene was introduced into the cells with an Ad vector. The results show that introduction of a HEX gene by using the Ad vector leads to enhanced induction of differentiation from the cells expressing an endodermal marker significantly to hepatocytes.

### (Example 6) Effect of induction of hepatocyte differentiation by HEX gene (18th day of culture)

In this Example, induction of differentiation from human iPS cells containing a HEX gene introduced therein using an Ad vector to hepatocytes will be described. The experimental scheme in this Example is shown in FIG. 14. 1) Construction of an Ad vector for introduction of HEX gene, 2) culture of human iPS cells and 3) introduction of HEX gene into human iPS cells were carried out in a manner similar to Example 1. 2) The human iPS cells were cultured by the following method:

### 2) Culture of human iPS cells

In this Example, human iPS cells (Tic (JCRB133D) were used. The cells were cultured similarly to (Example 1) until the 9th day after differentiation induction. The cells were then harvested with 0.0125% trypsin-0.01325 mM EDTA on the 9th day of culture and resuspended in HCMTM Hepatocyte Culture Medium (Lonza) containing SingleQuots® (Lonza), 10 ng/ml of fibroblast growth factor 4 (FGF4), 10 ng/ml of hepatocyte growth factor (HGF) (R&D Systems), 10 ng/ml of oncostatin M (R&D Systems) and 10⁻⁷ M dexamethasone (Sigma) added thereto, as described in Stem Cells, 26: 1117-27 (2008), and the suspension was inoculated on a type-I collagen (Nitta Gelatin)-coated cell culture plate (12 wells). The medium was exchanged once a day.

### (Test Example 6-1) Results obtained by immunostaining method

On the 18th day of culture, expression of respective genes was determined by an immunostaining method by using Alexa®594-labeled anti-albumin (ALB) antibody (manufactured by SIGMA), Alexa®594-labeled anti-drug-metabolizing enzyme cytochrome P450 3A4 (CYP3A4) antibody (manufactured by Santa Cruz) and Alexa®594-labeled anti-drug-metabolizing enzyme cytochrome P450 7A1 (CYP7A1) antibody (manufactured by Santa Cruz). Albumin, CYP3A4 and VYP7A1 were all hepatocyte markers. As shown in FIG. 15, it was found that all hepatocyte markers reacted significantly in the systems containing the HEX gene introduced with the Ad vector than in the systems without the gene introduced on the 18th of culture.

### (Example 7) Evaluation of functionality of HEX gene-introduced cells

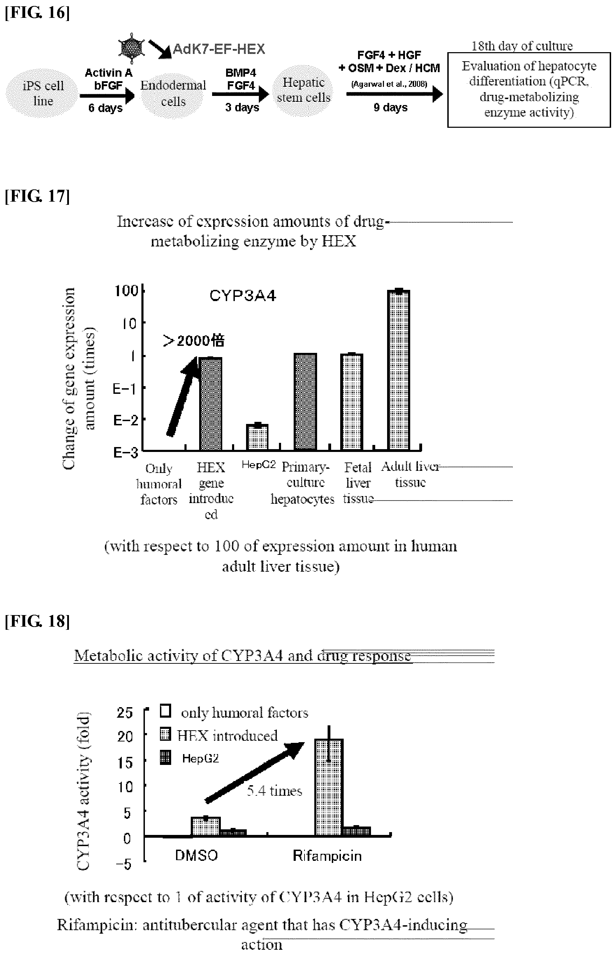

In this Example, induction of differentiation from human iPS cells containing a HEX gene introduced by using an Ad vector to hepatocytes will be described. The experimental scheme in this Example is shown in FIG. 16. 1) Construction of an Ad vector for introduction of HEX gene and 3) introduction of HEX gene into human iPS cells were carried out in a manner similar to Example 1, while 2) culture of human iPS cells was carried out in a manner similar to Example 6. Human iPS cells (Tic (JCRB1331)) were used in this Example.

### (Test Example 7-1) Results obtained by real-time PCR method

The expression amount of the drug-metabolizing enzyme cytochrome P450 3A4 (CYP3A4) in the cells on the 18th day of culture was determined by a real-time PCR method by using TaqMan® Gene Expression Assays (Applied Biosystems, catalog number: Hs00430021_m1 for CYP3A4). The expression amount was calculated, based on the standard (100) of the expression amounts of respective genes in Human Adult Liver Total RNA (Clontech, catalog number: 636531) (FIG. 17).

As a result, it was found that introduction of HEX gene with the Ad vector leads to drastic increase of the expression amount of CYP3A4 to the amount equivalent to that of fetal hepatocytes.

### (Test Example 7-2) Measurement of activity of drug-metabolizing enzyme cytochrome P450 3A4 (CYP3A4)

25 µM rifampicin (Sigma) or DMSO was added to human iPS cell-derived hepatocytes or human hepatoma cell line HepG2 on the 18th day after differentiation induction and the activity of CYP3A4 was determined by using P450-Glo™ CYP3A4 Assay Kit (Promega) after 72 hours. The activity was determined quantitatively by using a luminometer (Lumat LB 9507, Berthold).

It was found that the activity of CYP3A4 increased and drug response to rifampicin also increased in the system containing the HEX gene introduced with the Ad vector, compared to the system without the introduce gene on the 18th day of culture (FIG. 18)

### (Example 8) Induction of differentiation from iPS cell line that easily differentiates into endodermal cells to hepatocytes

In this Example, induction of differentiation from human iPS cells containing a HEX gene introduced by using an Ad vector to hepatocytes will be described. The experimental scheme in this Example is shown in FIG. 16. 1) Construction of an Ad vector for introduction of HEX gene and 3) introduction of HEX gene into human iPS cells were carried out in a manner similar to Example 1, while 2) culture of human iPS cells was carried out in a manner similar to Example 6. In this Example, human iPS cells (Tic (JCRB1331) and Dotcom (JCRB1327)) were used.

### (Test Example 8-1) Measurement of activity of drug-metabolizing enzyme cytochrome P450 3A4 (CYP3A4)

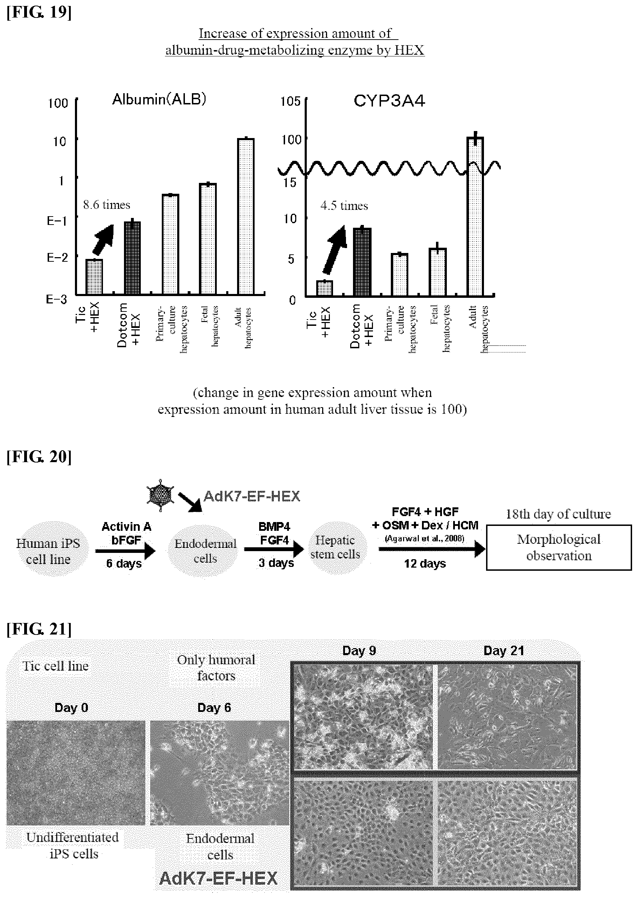

The activity of CYP3A4 was determined in a manner similar to Example 7-2. As a result, it was possible, by introducing the HEX gene with an Ad vector, to induce differentiation from a cell line Dotcom higher in the potential of differentiating into endodermal cells to hepatocytes that express albumin and GYP3A4 in greater amounts (FIG. 19).

### (Example 9) Morphological change during differentiation induction

In this Example, induction of differentiation from human iPS cells containing a HEX gene introduced by using an Ad vector to hepatocytes will be described. The experimental scheme in this Example is shown in FIG. 20. 1) Construction of an Ad vector for introduction of HEX gene and 3) introduction of HEX gene into human iPS cells were carried out in a manner similar to Example 1, while 2) culture of human iPS cells was carried out in a manner similar to Example 6. Human iPS cells (Tic (JCRB1331)) were used in this Example.

### (Test Example 9-1) Morphological observation

The shape of the cells on the 0th day of culture (undifferentiated cell), 6th day (endodermal cell), 9th day (hepatic stem cell) and 21st day was observed. As a result, the appearance of the cells changed from the shape of dense cells (0th day of culture) to the shape characteristic for the differentiated cells, in which the cellular density is low (6th day of culture). In addition, the cells in the system containing HEX gene introduced with an Ad vector changed into epithelial cell-like shape characteristic to hepatocytes over the period of 9th to 21st day of culture. Thus, the results of morphological observation also suggested that the differentiated cells induced by introduction of the HEX gene were hepatocytes (FIG. 21).

### (Example 10) Method of differentiation from human iPS cells to hepatocytes by using SOX17 and HEX genes in combination

In this Example, SOX17 and HEX genes were introduced into human iPS cells with an Ad vector and induction of differentiation from the gene-containing human iPS cells to hepatocytes will be described. The experimental scheme in this Example is shown in FIG. 22.

### 1) Construction of Ad vector for introduction of SOX17 gene

For introduction of the SOX17 gene, an Ad vector having a gene introduced in the E1-deficient region of E1-deficient type-5 Ad genome (pAdHM41-K7) and an EF-1α promoter in the region upstream thereof was prepared. The introduced gene was the SOX17 gene having the sequence of GenBank Accession No. NM_022454. The Ad vector for introduction of HEX gene was prepared in a manner similar to Example 1.

### 2) Culture of human iPS cells

Human iPS cells (201B7(JCRB)) were used in this Example. The cells were cultured similarly to the method of Example 1 until the 3rd day after differentiation induction.

### 3) Introduction of SOX17 gene into human iPS cells

After culture for 72 hours from induction of differentiation, the cells were infected with the AdK7 vector containing SOX17 gene inserted in step 1) at 3,000 VP/cell for 1.5 hours, and the medium was replaced with the medium 4 containing 50 ng/ml of activin A (R&D Systems) and 10 ng/ml of bFGF (R&D Systems) added thereto. The medium was then replaced with the medium 4 containing 50 ng/ml of Activin A (R&D Systems) and 10 ng/ml of bFGF (R&D Systems) every day.

### 4) Introduction of HEX gene into SOX17 gene-overexpressing cells

On the 5th day after induction of differentiation, the human iPS cells were separated with 0.0125% trypsin-0.01325 mM EDTA from the cell culture plate; trypsin was neutralized with 0.1mg/ml trypsin inhibitor; and the suspension was centrifuged from the medium 5 shown in Reference Example at 1,300 rpm for 3 minutes. The human iPS cells obtained after centrifugation was resuspended into the medium 5 containing 50 ng/ml of activin A (R&D Systems) and 10 ng/ml of bFGF (R&D Systems), inoculated in each well of a laminin (sigma)-coated cell culture plate (12 wells) at 5.0×10⁵ cell/well and cultured at 37°C. After culture for 24 hours, the cells were infected with the AdK7 vector containing the HEX gene inserted thereto at 3, 000 VP/cell for 1.5 hours, and the medium was replaced with the medium 5 containing 10 ng/ml of BMP4 (R&D Systems) and 10 ng/ml of FGF4 (R&D Systems). The medium was then exchanged with the medium 5 containing 10 ng/ml of BMP4 (R&D Systems) and 10 ng/ml of FGF4 (R&D Systems) once a day.

### (Test Example 10-1) Results obtained by immunostaining method

On the 9th and 12th days of culture, expression of respective genes was determined by an immunostaining method by using Alexa®594-labeled anti-α-fetoprotein (AFP) antibody (manufactured by Dako), Alexa®594-labeled anti-albumin (ALB) antibody (manufactured by SIGMA) and Alexa®488-labeled anti-CK7 antibody (manufactured by Invitrogen). AFP is a marker for hepatic stem cells, albumin, a marker for hepatocytes, and CK7, a marker for biliary epithelial cells. As shown in FIG. 23, on the 9th day of culture, the hepatic stem cell marker reacted significantly more in the cells overexpressing SOX17 and HEX genes, as introduced with the Ad vector, than in the cells overexpressing only the HEX gene introduced, but the biliary epithelial cell marker was not expressed in neither of the cells. On the 12th day of culture, the hepatocyte marker was found to react significantly more in the cells overexpressing SOX17 gene and HEX gene, as introduced with the Ad vector, than in the cells overexpressing only the HEX gene introduced.

### (Test Example 10-2) Results obtained by real-time PCR method

The expression amounts of the possible hepatic stem cell markers AFP and albumin in the cells on the 9the and 12th days of culture were determined by a real-time PCR method by using TaqMan® Gene Expression Assays (Applied Biosystems, catalog number: Hs01040607 _m1 for AFP and Hs00910225 _m for albumin). The expression amounts were calculated, based on the standard (100) of the expression amount of each gene in Human Fetal Liver Total RNA (Clontech, catalog number: 636540). The results show that introduction of SOX17 and HEX genes with the Ad vector leads to increased induction of differentiation from human iPS cells to hepatocytes, compared to the case when only the HEX gene was used (FIG. 24).

### (Example 11) Method of differentiation from human iPS cells to hepatocytes by HEX and HNF4A genes in combination

In this Example, induction of differentiation from human iPS cells containing HEX and HNF4A genes introduced by using an Ad vector to hepatocytes will be described. The experimental scheme in this Example is shown in FIG. 25.

### 1) Construction of Ad vector for introduction of HNF4A gene

An Ad vector having the gene introduced in the E1-deficient region of E1-deficient type-5 Ad genome (pAdHM41-K7) and an EF-1α promoter in the region upstream thereof was prepared. The introduced gene was the HNF4A gene having the sequence of GenBank Accession No. NM_000457. The Ad vector for introduction of HEX gene was prepared in a manner similar to Example 1.

### 2) Culture of human iPS cells

Human iPS cells (Tic (JCRB1331), Dotcom (JCRB1327), and 201B7) were used in this Example. The cells were cultured in a manner similar to Example 1 until the 9th day from induction of differentiation.

### 3) Introduction of HEX and HNF4A genes into human iPS cells

On the 9th day from induction of differentiation, the cells were infected with the AdK7 vector containing the HNF4A gene inserted in step 1) at 3,000 VP/cell for 1.5 hours, and the medium was replaced with the HCM™ medium (Lonza) containing 10 ng/ml of hepatocyte growth factor (HGF) (R&D Systems), 10 ng/ml of oncostatin M (R&D Systems), and 10⁻⁷ M dexamethasone (Sigma). The medium was then exchanged with the HCM™ medium (Lonza) containing 10 ng/ml of HGF (R&D Systems), 10 ng/ml of oncostatin M (R&D Systems) and 10⁻⁷ M dexamethasone (Sigma) once every two days.

### (Test Example 11-1) Results obtained by immunostaining method

On the 18th day of culture, expression of respective genes were determined by an immunostaining method by using Alexa®594-labeled anti-drug-metabolizing enzyme P450 2D6 (CYP2D6) antibody (manufactured by Santa Cruz), Alexa®594-labeled anti-drug-metabolizing enzyme P450 3A4 (CYP3A4) antibody (manufactured by Santa Cruz), and Alexa®594-labeled anti-drug-metabolizing enzyme P450 7A1 (CYP7A1) antibody (manufactured by Santa Cruz). CYP2D6, CYP3A4 and CYP7A1 are all hepatocyte markers. As shown in FIG. 26, on 18th day of culture, all hepatocyte markers were found to react significantly more in the cells overexpressing HEX gene and HNF4A gene, as introduced with the Ad vector, than in the cells overexpressing only the HEX gene introduced,

### (Test Example 11-2) Results obtained by real-time PCR method

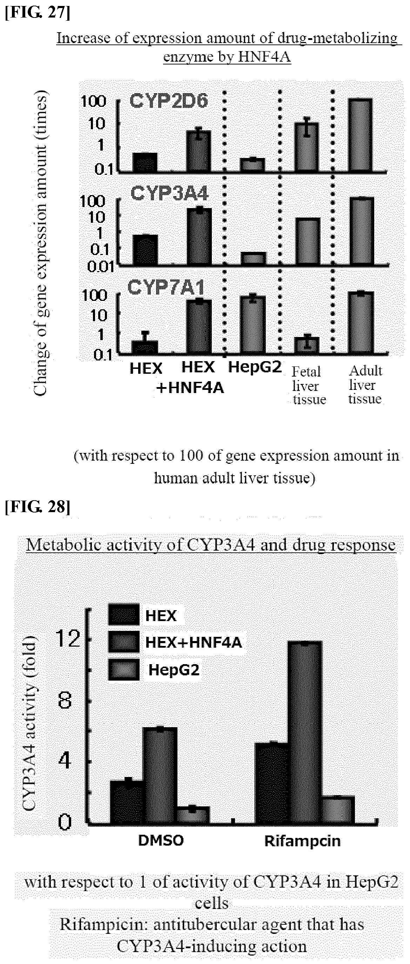

The expression amounts of the possible hepatocyte markers CYP2D6, CYP3A4 and CYP7A1 in the cells on the 18th day of culture were determined by a real-time PCR method by using TaqMan® Gene Expression Assays (Applied Biosystems, catalog number: Hs02576168_g1 for CYP2D6, Hs00430021_m1 for GYP3A4 and Hs00167982_m1 for CYP7A1). The expression amounts were calculated, based on the standard (100) of the expression amount of each gene in Human Adult Liver Total RNA (Clontech, catalog number: 636531). The results show that introduction of HEX and NF4A genes with the Ad vector leads to drastically increase of the expression amount of CYP2D6, CYP3A4 and CYP7A1, compared to the case when only the HEX gene was used, and thus it was possible to obtain hepatocytes higher in the expression amounts of medicine metabolizing enzymes than normal cells (FIG. 27).

### (Test Example 11-3) Measurement of activity of drug-metabolizing enzyme P450 3A4

25 µM rifampicin (Sigma) or DMSO was added to hepatocyte or human hepatoma cell line HepG2 derived from the human iPS cells on the 18th day from induction of differentiation and the activity of CYP3A4 was determined by using P450-G1o™ CYP3A4 Assay Kit (Promega) after 72 hours. The activity was determined quantitatively by using a luminometer (Lumat LB 9507, Berthold). The results showed that introduction of HEX and HNF4A genes with the Ad vector leads to increase of the activity of CYP3A4 and also increase in drug response to rifampicin, compared to the case where only the HEX gene was used. It was thus possible to obtain hepatocytes higher in the activity of drug-metabolizing enzyme than normal cells, by introduction of the HEX and HNF4A genes with the Ad vector by the method above (FIG. 28).

### (Example 12) Method of differentiation from human iPS cells to hepatocytes by introducing SOX17, HEX and HNF4A genes in combination

In this Example, induction of differentiation to hepatocytes containing SOX17, HEX and HNF4A genes introduced as needed by using an Ad vector will be described. The experimental scheme in this Example is shown in FIG. 29.

### 1) Ad vector for introduction of SOX17, HEX and HNF4A genes

An Ad vector similar to that in Example 1 was used.

### 2) Culture of human iPS cells

Human iPS cells (Tic (JCRB1331)) were used in this Example. The cells were cultured in a manner similar to Example 1 until the 3rd day after induction of differentiation.

### 3) Introduction of SOX17 gene

On the 3rd day from induction of differentiation, the cells were infected with the AdK7 vector containing the SOX17 gene inserted thereto at 3,000 VP/cell for 1.5 hours, and the medium was replaced with the medium 4 containing 50 ng/ml of activin A (R&O Systems) and 10 ng/ml of bFGF (R&D Systems). The medium was then replaced with the medium 4 containing 50 ng/ml of activin A (R&D Systems) and 10 ng/ml of bFGF (R&D Systems) once a day. The cells without SOX17 gene introduced were uses as a control group.

### 4) Introduction of HEX gene

On the 6th day after induction of differentiation, the cells were separated from the cell culture plate with 0.0125% trypsin-0.01325 mM EDTA; trypsin was neutralized with 0.1 mg/ml trypsin inhibitor; and the cells were centrifuged at 1,300 rpm for 3 minutes twice by using the medium 5 shown in Reference Example. The human iPS cells obtained after centrifugation were resuspended in the medium 5 containing 50 ng/ml of activin A (R&D Systems) and 10 ng/ml of bFGF (R&D Systems) added thereto, inoculated in each well on a laminin (sigma)-coated cell culture plate (12 wells) in an amount of 5.0×10⁵ cell/well and cultured at 37°C. After 24 hours, the cells were infected with the AdK7 vector containing the inserted HEX gene at 3,000 VP/cell for 1.5 hours and the medium was replaced with the medium 5 containing 10 ng/ml of BMP4 (R&D Systems) and 10 ng/ml of FGF4 (R&D Systems). The medium was then exchanged with the medium 5 containing 10 ng/ml of BMP4 (R&D Systems) and 10 ng/ml of FGF4 (R&D Systems) added thereto once a day. The cells without the HEX gene introduced were used as a control group.

### 5) Introduction of HNF4A gene

On the 9th day after induction of differentiation, the cells were infected with the AdK7 vector containing the HNF4A gene inserted in step 1) above at 3,000 VP/cell for 1.5 hours and the medium was replaced with the HCM™medium (Lonza) containing 10 ng/ml of HGF (R&D Systems), 10 ng/ml of oncostatin M (R&D Systems) and 10⁻⁷ M dexamethasone (Sigma) added thereto. The medium was then exchanged with the HCM™medium (Lonza) containing 10 ng/ml of HGF (R&D Systems), 10 ng/ml of oncostatin M (R&D Systems) and 10⁻⁷ M dexamethasone (Sigma) added thereto once every two days. The cells without the HNF4A gene introduced were used as a control group.

The combination of the genes introduced is shown in Table 1.

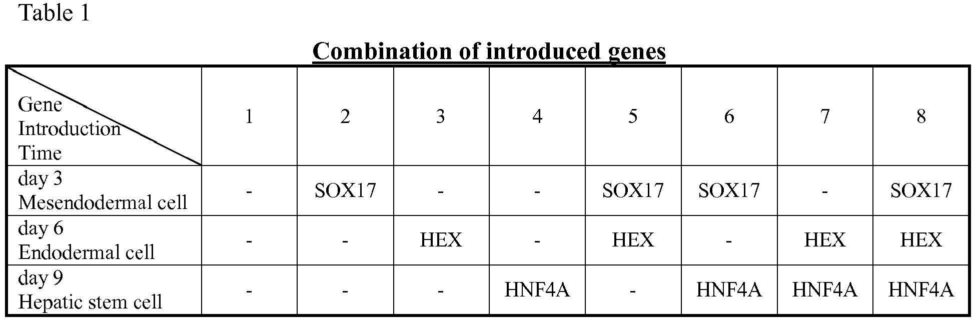

### (Test Example 12) Results obtained by real-time PCR method

On the 18th day of culture, the expression amounts of possible hepatocyte markers ALB, GYP2D6, CYP3A4 and CYP7A1 were determined by a real-time PCR method by using TaqMan® Gene Expression Assays (Applied Biosystems, catalog number: Hs00910225_m for albumin (ALB), Hs02576168_g1 for GYP2D6, Hs00430021_m1 for CYP3A4 and Hs00167982_m1 for CYP7A1). The expression amounts were calculated, based on the standard (100) of the expression amounts of respective genes in human adult liver total RNA (Clontech, catalog number 636531). The results are shown in FIG. 30. The number on the abscissa in FIG. 30 represents the combination of genes shown in Table 1. As a result, albumin, CYP2D6, CYP3A4, CYP7A1 were expressed in drastically increased amounts in the cells containing all three SOX17, HEX and HNF4A genes introduced by using an Ad vector, compared to the case when other combinations of genes were introduced, and thus, it was possible to obtain hepatocytes that give the drug-metabolizing enzyme in an amount greater than normal cells.

### Industrial Applicability

As described above in detail, it was shown that it is possible by using the Ad vector according to the present invention to introduce genes associated with induction of differentiation of pluripotent stem cells such as ES or iPS cells effectively and to induce differentiation to hepatocytes. Specifically, it is possible to induce differentiation to hepatocytes effectively by introducing one or more genes selected from HEX, HNF4A, HNF6 and SOX17 genes into stem cells such as ES or iPS cells. It was found that it is possible to induce differentiation from stem cells to hepatocytes effectively, especially by introducing a HEX gene into stem cells. It was also found that it is possible to induce of differentiation from stem cells to hepatocytes more effectively by introducing for example SOX17 gene and/or HNF4A gene additionally, as needed, according to progress of cell differentiation.

It is possible for example to perform evaluation of pharmaceutical toxicity and pharmacokinetics by using the hepatocytes prepared by the method of differentiation according to the present invention. Specifically, it is possible to predict toxicity of a candidate drug compound in the body previously by adding the candidate drug compound to the hepatocytes prepared according to the present invention and analyzing the fluctuation in expression of hepatotoxicity markers. It is also possible to predict the pharmacokinetics (metabolites) of a candidate drug compound previously by adding the candidate drug compound to the hepatocytes prepared according to the present invention and analyzing the metabolites of the compound. It is possible in this way to screen rapidly candidate drug compounds that are to be eliminated because of their problems in toxicity and pharmacokinetics, thus accelerating drug development.

If it is possible to induce differentiation from pluripotent stem cells such as ES or iPS cells to hepatocytes by the method according to the present invention, it becomes possible to reconstruct the hepatocyte structure in vitro and further it may be possible to prepare mature hepatocytes and further the liver itself by differentiation induction. It is possible by using the Ad vector according to the present invention to induce differentiation from various ES or iPS cells to hepatocytes without preparation of a stabilized strain expressing a particular gene. It is thus possible to prepare the individual liver specific to the patient rapidly if iPS cells are available. In addition, the method according to the present invention may pave a way to treatment by regenerative medicine of diseases that can be treated only with organ transplantation traditionally and thus, it is quite useful.

## Claims

1. An in vitro method of differentiation from human stem cells to hepatocytes, comprising the steps
(1) providing embryonic stem cells (ESC) or induced pluripotent stem cells (iPS),
(2) inducing the pluripotent stem cells by incubation with a humoral differentiation-inducing factor selected from the group consisting of Activin and bFGF,
(3) transducing the pluripotent stem cells comprising the mesendodermal cells, the endodermal cells and hepatic stem cells with one or more genes selected from HEX, HNF4A, HNF6 and SOX17 genes into stem cells using an adenovirus vector and
(4) culturing the cells together with hepatozyte growth factor (HGF) to obtain hepatocytes.

2. The method of differentiation according to claim 1 wherein at least the HEX gene is introduced and additionally the HNF4A gene and/or the SOX17 gene are introduced.

3. The method of differentiation according to claim 2, wherein the SOX17 gene and then the HEX gene are introduced into the stem cells.

4. The method of differentiation according to claim 2 or 3, wherein the HEX gene and then the HNF4A gene are introduced into the stem cells.

5. A method of differentiation from human stem cells to hepatocytes, comprising the steps of:
1) introducing an HEX gene into an adenovirus vector;
2) introducing the HEX gene into cells by bringing the adenovirus vector containing the gene introduced in step 1) into contact with stem cells; and
3) culturing the stem cells containing the gene introduced therein.

6. The method of differentiation from stem cells to hepatocytes according to claim 5, comprising, before the steps 1) to 3) above, the following steps of:
a) introducing an SOX17 gene into an adenovirus vector;
b) introducing the SOX17 gene into cells by bringing the adenovirus vector containing the gene introduced in step a) into contact with stem cells; and
c) culturing the stem cells containing the gene introduced therein.

7. The method of differentiation from stem cells to hepatocytes according to claim 5 or 6, comprising, after the step of culturing the stem cells containing the HEX gene introduced in step 3) of claim 5 or step c) of claim 6, a step of introducing an HNF4A gene into the cells additionally by bringing the cultured cell into contact with an adenovirus vector containing the HNF4A gene introduced therein.

8. The method of differentiation according to claim 7, wherein the stem cells are treated with a humoral differentiation-inducing factor before the step of introducing the HEX or HNF4A gene into cells by bringing the adenovirus vector containing the HEX or HNF4A gene introduced into contact with stem cells.

9. The method of differentiation according to any one of claims I or 8, wherein the stem cell is an embryonic stem cell or an induced pluripotent stem cell.

## Patentansprüche

1. In-vitro-Verfahren zur Differenzierung von humanen Stammzellen zu Hepatozyten, umfassend die Schritte
(1) Bereitstellen von embryonalen Stammzellen (ESC) oder induzierten pluripotenten Stammzellen (iPS),
(2) Induzieren der pluripotenten Stammzellen durch Inkubation mit einem humoralen differenzierungsinduzierenden Faktor, ausgewählt aus der Gruppe bestehend aus Activin und bFGF,
(3) Transduktion der pluripotenten Stammzellen, die die mesendodermalen Zellen, die endodermalen Zellen und hepatischen Stammzellen umfassen, mit einem oder mehreren Genen, ausgewählt aus den Genen HEX, HNF4A, HNF6 und SOX17, in Stammzellen unter Verwendung eines Adenovirusvektors, und
(4) Kultivieren der Zellen zusammen mit Hepatozytenwachstumsfaktor (HGF), um Hepatozyten zu erhalten.

2. Verfahren zur Differenzierung nach Anspruch 1, wobei wenigstens das HEX-Gen eingeführt wird und zusätzlich das HNF4A-Gen und/oder das SOX17-Gen eingeführt werden.

3. Verfahren zur Differenzierung nach Anspruch 2, wobei das SOX17-Gen und dann das HEX-Gen in die Stammzellen eingeführt werden.

4. Verfahren zur Differenzierung nach Anspruch 2 oder 3, wobei das HEX-Gen und dann das HNF4A-Gen in die Stammzellen eingeführt werden.

5. Verfahren zur Differenzierung von humanen Stammzellen zu Hepatozyten, umfassend die Schritte:
1) Einführen eines HEX-Gens in einen Adenovirusvektor;
2) Einführen des HEX-Gens in Zellen durch Inkontaktbringen des Adenovirusvektors, der das in Schritt 1) eingeführte Gen enthält, mit Stammzellen; und
3) Kultivieren der Stammzellen, die das darin eingeführte Gen enthalten.

6. Verfahren zur Differenzierung von Stammzellen zu Hepatozyten nach Anspruch 5, umfassend vor den obigen Schritten 1) bis 3) die folgenden Schritte:
a) Einführen eines SOX17-Gens in einen Adenovirusvektor;
b) Einführen des SOX17-Gens in Zellen durch Inkontaktbringen des Adenovirusvektors, der das in Schritt a) eingeführte Gen enthält, mit Stammzellen; und
c) Kultivieren der Stammzellen, die das darin eingeführte Gen enthalten.

7. Verfahren zur Differenzierung von Stammzellen zu Hepatozyten nach Anspruch 5 oder 6, umfassend nach dem Kultivieren der Stammzellen, die das in Schritt 3) von Anspruch 5 oder Schritt c) von Anspruch 6 eingeführte HEX-Gen enthalten, einen Schritt des Einführens eines HNF4A-Gens in die Zellen zusätzlich durch Inkontaktbringen der kultivierten Zelle mit einem Adenovirusvektor, der das darin eingeführte HNF4A-Gen enthält.

8. Verfahren zur Differenzierung nach Anspruch 7, wobei die Stammzellen vor dem Schritt des Einführens des HEX- oder HNF4A-Gens in Zellen mit einem humoralen Differenzierungs-induzierenden Faktor behandelt werden, indem Adenovirusvektor, der das eingeführte HEX- oder HNF4A-Gen enthält, mit Stammzellen in Kontakt gebracht wird.

9. Verfahren zur Differenzierung nach einem der Ansprüche 1 oder 8, wobei die Stammzelle eine embryonale Stammzelle oder eine induzierte pluripotente Stammzelle ist.

## Revendications

1. Procédé in vitro de différenciation de cellules souches humaines en hépatocytes, comprenant les étapes consistant à
(1) fournir des cellules souches embryonnaires (CSE) ou des cellules souches pluripotentes induites (SPi),
(2) induire les cellules souches pluripotentes par incubation avec un facteur humoral induisant la différenciation, choisi dans l'ensemble constitué par l'activine et le bFGF,
(3) transduire les cellules souches pluripotentes, comprenant les cellules mésendodermiques, les cellules endodermiques et des cellules souches hépatiques, avec un ou plusieurs gène(s) choisi(s) parmi les gènes HEX, HNF4A, HNF6 et SOX17 dans des cellules souches en utilisant un vecteur adénoviral et
(4) cultiver les cellules conjointement avec du facteur de croissance des hépatocytes (HGF) pour obtenir des hépatocytes.

2. Procédé de différenciation selon la revendication 1, dans lequel on introduit au moins le gène HEX et on introduit en outre le gène HNF4A et/ou le gène SOX17.

3. Procédé de différenciation selon la revendication 2, dans lequel on introduit dans les cellules souches le gène SOX17 et ensuite le gène HEX.

4. Procédé de différenciation selon la revendication 2 ou 3, dans lequel on introduit dans les cellules souches le gène HEX et ensuite le gène HNF4A.

5. Procédé de différenciation de cellules souches humaines en hépatocytes, comprenant les étapes consistant à :
1) introduire un gène HEX dans un vecteur adénoviral ;
2) introduire le gène HEX dans des cellules en mettant en contact avec des cellules souches le vecteur adénoviral contenant le gène introduit dans l'étape 1) ; et
3) cultiver les cellules souches contenant le gène introduit dans celles-ci.

6. Procédé de différenciation de cellules souches en hépatocytes selon la revendication 5, comprenant, avant les étapes 1) à 3) ci-dessus, les étapes suivantes consistant à :
a) introduire un gène SOX17 dans un vecteur adénoviral ;
b) introduire le gène SOX17 dans des cellules en mettant en contact avec des cellules souches le vecteur adénoviral contenant le gène introduit dans l'étape a) ; et
c) cultiver les cellules souches contenant le gène introduit dans celles-ci.

7. Procédé de différenciation de cellules souches en hépatocytes selon la revendication 5 ou 6, comprenant, après l'étape consistant à cultiver les cellules souches contenant le gène HEX introduit dans l'étape 3) de la revendication 5 ou l'étape c) de la revendication 6, une étape consistant à introduire en outre dans les cellules un gène HNF4A en mettant la cellule cultivée en contact avec un vecteur adénoviral contenant le gène HNF4A introduit dans celui-ci.

8. Procédé de différenciation selon la revendication 7, dans lequel on traite les cellules souches par un facteur humoral induisant la différenciation, avant l'étape consistant à introduire le gène HEX ou HNF4A dans des cellules en mettant en contact avec des cellules souches le vecteur adénoviral contenant le gène HEX ou HNF4A introduit.

9. Procédé de différenciation selon l'une quelconque des revendications 1 et 8, dans lequel la cellule souche est une cellule souche embryonnaire ou une cellule souche pluripotente induite.
